## (19) Europäisches Patentamt
## European Patent Office
## Office européen des brevets

(11) **EP 0 858 478 B1**

(12) **EUROPÄISCHE PATENTSCHRIFT**

(45) Veröffentlichungstag und Bekanntmachung des Hinweises auf die Patenterteilung:
**19.01.2000 Patentblatt 2000/03**

(21) Anmeldenummer: **96934813.5**

(22) Anmeldetag: **25.10.1996**

(51) Int Cl.[7]: **C08J 9/30**, C08L 33/02, A61L 15/00

(86) Internationale Anmeldenummer:
**PCT/EP96/04644**

(87) Internationale Veröffentlichungsnummer:
**WO 97/17397 (15.05.1997 Gazette 1997/21)**

(54) **WASSERABSORBIERENDE, SCHAUMFÖRMIGE, VERNETZTE HYDROGEL-POLYMERE**

WATER-ABSORBENT CELLULAR CROSSLINKED HYDROGEL POLYMERS

POLYMERES HYDROGELS ABSORBANT L'EAU, EN MOUSSE, RETICULES

(84) Benannte Vertragsstaaten:
**DE ES FR GB IT SE**

(30) Priorität: **03.11.1995 DE 19540951**

(43) Veröffentlichungstag der Anmeldung:
**19.08.1998 Patentblatt 1998/34**

(73) Patentinhaber: **BASF AKTIENGESELLSCHAFT 67056 Ludwigshafen (DE)**

(72) Erfinder:
- **HÄHNLE, Hans-Joachim**
  **D-67435 Neustadt (DE)**
- **WALTER, Manfred**
  **D-67346 Speyer (DE)**
- **TROPSCH, Jürgen**
  **D-67354 Römerberg (DE)**
- **SCHORNICK, Gunnar**
  **D-67271 Neuleiningen (DE)**
- **ANSTOCK, Thomas**
  **D-67256 Weisenheim (DE)**

(56) Entgegenhaltungen:
**DE-A- 4 418 818**

Bemerkungen:
Die Akte enthält technische Angaben, die nach dem Eingang der Anmeldung eingereicht wurden und die nicht in dieser Patentschrift enthalten sind.

## Beschreibung

Die Erfindung betrifft wasserabsorbierende, schaumförmige, vernetzte Polymerisate, Verfahren zur ihrer Herstellung und ihre Verwendung in Sanitärartikeln, die zur Absorption von Körperflüssigkeiten und in Verbandmaterial zur Abdeckung von Wunden eingesetzt werden.

Wasserabsorbierende, vernetzte Polymerisate werden als Superabsorber oder superabsorbierende Polymere bezeichnet, weil sie in der Lage sind, ein Vielfaches ihres Eigengewichtes an wäßrigen Flüssigkeiten unter Ausbildung von Hydrogelen aufzunehmen. In der Praxis werden Superabsorber beispielsweise in Windeln zur Absorption von Urin eingesetzt. Die Superabsorber haben die Eigenschaft, die absorbierte Flüssigkeit auch unter mechanischer Belastung zurückzuhalten.

Um die anwendungstechnischen Eigenschaften von Superabsorbern zu variieren, sind zwei unterschiedliche Typen von Schäumen bekannt, (1) Mischungen, die Superabsorber in einer geschäumten Matrix enthalten, und (2) Schäume, die aus einem superabsorbierenden Material bestehen.

Ein unter die Kategorie (1) fallender Schaum wird beispielsweise aus einer Mischung hergestellt, die einerseits Komponenten für die Bildung eines Polyurethanschaums und andererseits polymerisierbare Monomere, einen Vernetzer und einen Polymerisationsinitiator zur Herstellung eines Superabsorbers enthält. Aus einer solchen Mischung wird in einer Polykondensationsreaktion aus den Polyurethankomponenten der Schaum gebildet, der den durch Polymerisation der Monomeren entstehenden Superabsorber in Form eines interpenetrierenden Netzwerkes enthält, vgl. US-A-4 725 628, US-A-4 725 629 und US-A-4 731 391.

Aus der US-A-4 985 467 ist ein Polyurethanschaum bekannt, der einen Superabsorber chemisch gebunden enthält. Außerdem sind Kombinationen von Latex-Schäumen bekannt, in die nach dem Schäumprozeß superabsorbierende, feinteilige Materialien eingearbeitet werden, vgl. EP-A-427 219 und US-A-4 990 541.

Zu der Kategorie (2) von Schäumen gehören beispielsweise solche Produkte, die dadurch erhalten werden, daß man einen vorgefertigten Superabsorber in einem Extruder mit einer Polyhydroxyverbindung und einem Treibmittel bei erhöhter Temperatur mischt. Beim Auspressen der Mischung aus dem Extruder bildet sich der Schaum. Verfahren dieser Art sind beispielsweise in der US-A-4 394 930, US-A-4 415 388 und GB-A-2 136 813 beschrieben.

Aus US-A-4 529 739 und US-A-4 649 154 sind Verfahren zur Herstellung von Schäumen bekannt, wobei man ein wasserquellbares, COOH-Gruppen tragendes Material mit einem Treibmittel aufschäumt, das in einer Neutralisierungsreaktion mit den COOH-Gruppen des Polymeren das Treibgas freisetzt.

Gemäß den Angaben in der WO-A-94/22502 werden superabsorbierende Schäume auf Basis von vernetzten, teilweise neutralisierten Polycarboxylaten dadurch hergestellt, daß man eine Monomermischung mit einem in Wasser unlöslichen Treibmittel schäumt, das einen Siedepunkt unterhalb von 50°C hat, und den Schaum praktisch gleichzeitig mit dem Schäumen auspolymerisiert.

Aus der EP-A-04 21 264 ist die Herstellung schaumartiger Superabsorber bekannt, wobei man eine wäßrige Monomermischung, die eine Ölphase emulgiert enthält, polymerisiert. Das Öl wirkt hierbei als Platzhalter für die späteren Poren des Schaums und wird nach beendeter Polymerisation beim Trocknen des schaumförmigen Materials durch Verdampfen entfernt.

Aus der WO-A-88/09801 ist bekannt, daß man hydrophile Polymere, z.B. Polynatriumacrylat in Gegenwart von Vernetzern wie Polyepoxiden und Treibmitteln durch Erwärmen zu einem schaumförmigen Superabsorber verarbeiten kann.

Zur Herstellung schaumförmiger Superabsorber ist außerdem eine Arbeitsweise bekannt, bei der man Carbonate, Hydrogencarbonate oder Kohlendioxid als Treibmittel zu einer Mischung aus Carboxylgruppen tragenden Monomeren, Vernetzungsmittel und Polymerisationsinitiator zusetzt, wobei zeitgleich mit der Zugabe des Treibmittels oder kurz darauf die Polymerisation der Monomeren gestartet wird. Der Superabsorber erhält durch das bei der Neutralisationsreaktion gebildete Kohlendioxid eine Schaumstruktur, vgl. US-A-4 808 637. Nach dem aus der WO-A-95/02002 bekannten Verfahren wird ein schaumförmiger Superabsorber im Anschluß an die Herstellung mit einer oder mehreren zur nachträglichen Oberflächenvernetzung reaktionsfähigen Verbindungen versetzt und auf eine Temperatur von 100 bis 300°C erhitzt.

Bei den oben beschriebenen Verfahren zur Herstellung von superabsorbierenden Schäumen erfolgen die Bildung des Schaums und die Polymerisation entweder synchron oder nur unwesentlich zeitlich versetzt. Die noch nicht auspolymerisierten Schäume haben nur eine geringe Standzeit, meistens beträgt sie nur wenige Minuten. Nachteilig bei den oben angegebenen Verfahren ist beispielsweise der Einsatz größerer Treibmittelmengen, insbesondere der Einsatz von FCKW im Fall der WO-A-94/22502.

Der Erfindung liegt die Aufgabe zugrunde, schaumförmige Superabsorber zur Verfügung zu stellen.

Die Aufgabe wird erfindungsgemäß gelöst mit wasserabsorbierenden, überwiegend offenzelligen schaumförmigen, vernetzten Polymerisaten, die erhältlich sind durch

(I) Schäumen einer polymerisierbaren wäßrigen Mischung, die

(a) 10 bis 80 Gew.-% Säuregruppen enthaltende monoethylenisch ungesättigte Monomere, die zu mindestens 50 Mol-% neutralisiert sind,

(b) gegebenenfalls bis zu 50 Gew.-% andere monoethylenisch ungesättigte Monomere,

(c) 0,001 bis 5 Gew.-% Vernetzer,

(d) Initiatoren,

(e) 0,1 bis 20 Gew.-% mindestens eines Tensids,

(f) gegebenenfalls mindestens einen Lösevermittler und

(g) gegebenenfalls Verdicker, Schaumstabilisatoren, Polymerisationsregler, Füllstoffe und/oder Zellkeimbildner

enthält, wobei das Schäumen durch Dispergieren von feinen Blasen eines gegenüber Radikalen inerten Gases erfolgt, und

(II) Polymerisieren der geschäumten Mischung unter Bildung eines schaumförmigen Hydrogels und Einstellen des Wassergehalts des Polymerisats auf 1 bis 45 Gew.-%.

Gegenstand der Erfindung ist außerdem ein Verfahren zur Herstellung von wasserabsorbierenden, schaumförmigen, vernetzten Polymerisaten, wobei man eine polymerisierbare wäßrige Mischung aus

(a) 10 bis 80 Gew.-% Säuregruppen enthaltenden monoethylenisch ungesättigten Monomeren, die zu mindestens 50 Mol-% neutralisiert sind,

(b) gegebenenfalls bis zu 50 Gew.-% anderen monoethylenisch ungesättigten Monomeren,

(c) 0,001 bis 5 Gew.-% Vernetzer,

(d) Initiatoren,

(e) 0,1 bis 20 Gew.-% mindestens einem Tensid,

(f) gegebenenfalls mindestens einem Lösevermittler und

(g) gegebenenfalls Verdickern, Schaumstabilisatoren, Polymerisationsreglern, Füllstoffen und/oder Zellkeimbildnern

in einer ersten Verfahrensstufe durch Dispergieren von feinen Blasen eines gegenüber Radikalen inerten Gases schäumt und den so erhaltenen Schaum in einer zweiten Verfahrensstufe unter Bildung eines schaumförmigen Hydrogels polymerisiert und den Wassergehalt des schaumförmigen Hydrogels auf 1 bis 45 Gew.-% einstellt.

Erfindungsgemäß wird eine polymerisierbare wäßrige Mischung zu einem Schaum verarbeitet, der verarbeitungsstabil ist und beliebig geformt werden kann. Die polymerisierbare wäßrige Mischung enthält als Komponenten (a) Säuregruppen enthaltende monoethylenisch ungesättigte Monomere, die zu mindestens 50 Mol-% neutralisiert sind. Solche Monomere sind beispielsweise monoethylenisch ungesättigte $C_3$- bis $C_{25}$-Carbonsäuren oder Anhydride, beispielsweise Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure.

Als Monomere der Gruppe (a) kommen außerdem monoethylenisch ungesättigte Sulfonsäuren in Betracht, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Vinylphosphonsäure, Allylphosphonsäure und 2-Acrylamido-2-methylpropansulfonsäure. Die Monomeren können allein oder in Mischung untereinander bei der Herstellung der Superabsorber eingesetzt werden. Bevorzugt eingesetzte Monomere der Gruppe (a) sind Acrylsäure, Methacrylsäure, Vinylsulfonsäure, Acrylamidopropansulfonsäure oder Mischungen dieser Säuren, z.B. Mischungen aus Acrylsäure und Methacrylsäure, Mischungen aus Acrylsäure und Acrylamidopropansulfonsäure oder Mischungen aus Acrylsäure und Vinylsulfonsäure.

Die Monomeren sind zu mindestens zu 50 Mol-% neutralisiert. Zur Neutralisation verwendet man beispielsweise Alkalimetallbasen oder Ammoniak bzw. Amine. Zur Neutralisation setzt man vorzugsweise Natronlauge oder Kalilauge ein. Die Neutralisation kann jedoch auch mit Hilfe von Natriumcarbonat, Natriumhydrogencarbonat, Kaliumcarbonat oder Kaliumhydrogencarbonat oder anderen Carbonaten oder Hydroygencarbonate oder Ammoniak vorgenommen werden. Die Säuregruppen der Monomeren werden vorzugsweise zu mindestens 65 Mol-% neutralisiert.

Die polymerisierbare wäßrige Mischung kann gegebenenfalls Monomere der Gruppe (b) enthalten. Hierunter sollen andere monoethylenisch ungesättigte Monomere verstanden werden, die mit den Monomeren (a) und (c) copolymerisierbar sind. Hierzu gehören beispielsweise die Amide und Nitrile von monoethylenisch ungesättigten Carbonsäuren, z.B. Acrylamid, Methacrylamid und N-Vinylformamid, Acrylnitril und Methacrylnitril, Dialkyldiallylammoniumhalogenide wie Dimethyldiallylammoniumchlorid, Diethyldiallylammoniumchlorid, Allylpiperidiniumbromid, N-Vinylimidazole wie z.B. N-Vinylimidazol, 1-Vinyl-2-methylimidazol und N-Vinylimidazoline wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation eingesetzt werden können. Außerdem eignen sich Dialkylaminoalkylacrylate und Dialkylaminoalkylmethacrylate, z.B. Dimethylaminoethylacrylat, Dimethylaminoethylmethacrylat, Diethylaminoethylacrylat und Diethylaminoethylmethacrylat. Die basischen Estern werden vorzugsweise in quaternisierter Form oder als Salz eingesetzt. Weitere geeignete Verbindungen der Gruppe (b) sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z.B. Ethylvinylether oder Butylvinylether, Ester von monoethylenisch ungesättigten $C_3$- bis $C_6$-Carbonsäuren, z.B. Ester aus einwertigen $C_1$- bis $C_{18}$-Alkoholen und Acrylsäure, Methacrylsäure oder Maleinsäure, Halbester von Maleinsäure, z.B. Maleinsäuremonomethylester und Hydroxyalkylester der genannten monoethylenisch ungesättigten Carbonsäuren, z.B. 2-Hydroxyethylacrylat, Hydroxypropylacrylat, Hydroxybutylacrylat, Hydroxyethylmethacrylat, Hydroxypropylmethacrylat und Hydroxybutylmethacrylat, N-Vinyllactame wie N-Vinylpyrrolidon oder N-Vinylcaprolactam, Acrylsäure- und Methacrylsäureester von alkoxylierten einwertigen, gesättigten Alkoholen, z.B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_N$) der Polyalkylenglykole beispielsweise bis zu 2000 betragen können. Weiterhin geeignete Monomere der Gruppe (b) sind alkylsubstituierte Styrole wie Ethylstyrol oder tert. Butylstyrol. Die Monomeren der Gruppe (b) können auch in Mischung bei der Copolymerisation mit den anderen Monomeren eingesetzt werden, z.B. Mischungen aus Vinylacetat und 2-Hydroxyethylacrylat in beliebigem Verhältnis.

Die Monomeren der Gruppe (c) haben mindestens 2 ethylenisch ungesättigte Doppelbindungen. Beispiele für solche Monomere, die bei Polymerisationsreaktionen üblicherweise als Vernetzer eingesetzt werden, sind N,N'-Methylen-bisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Propylenglykoldiacrylat, Butandioldiacrylat, Hexandioldiacrylat, Hexandioldimethacrylat, Diacrylate und Dimethacrylate von Block-copolymerisaten aus Ethylenoxid und Propylenoxid, zweifach bzw. dreifach mit Acrylsäure oder Methacrylsäure veresterte mehrwertige Alkohole, wie Glycerin oder Pentaerythrit, Triallylamin, Tetraallylethylendiamin, Divinylbenzol, Diallylphthalat, Polyethylenglykoldivinylether von Polyethylenglykolen eines Molekulargewichts von 106 bis 4000, Trimethylolpropandiallylether, Butandioldivinylether, Pentaerythritttriallylether und/oder Divinylethylenharnstoff. Vorzugsweise setzt man wasserlösliche Vernetzer ein, z.B. N,N'-Methylen-bisacrylamid, Polyethylenglykoldiacrylate und Polyethylenglykoldimethacrylate, die sich von Additionsprodukten von 2 bis 400 Mol Ethylenoxid an 1 Mol eines Diols oder Polyols ableiten, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an ein Mol Glycerin, Pentaerythrittriallylether und/oder Divinylharnstoff.

Als Vernetzer kommen außerdem Verbindungen in Betracht, die mindestens eine polymerisierbare ethylenisch ungesättigte Gruppe und mindestens eine weitere funktionelle Gruppe enthalten. Die funktionelle Gruppe dieser Vernetzer muß in der Lage sein, mit den funktionellen Gruppen, im wesentlichen den Carboxylgruppen oder Sulfonsäuregruppen der Monomeren (a) zu reagieren. Geeignete funktionelle Gruppen sind beispielsweise Hydroxyl-, Amino-, Epoxi- und Aziridinogruppen.

Als Vernetzer kommen außerdem solche Verbindungen in Betracht, die mindestens zwei funktionelle Gruppen tragen, die mit Carboxyl- und Sulfonsäuregruppen der eingesetzten Monomeren der Gruppe (a) reagieren können. Die geeigneten funktionellen Gruppen wurden bereits oben genannt, d.h. Hydroxyl-, Amino-, Epoxi-, Isocyanat-, Ester-, Amide- und Aziridinogruppen. Beispiele für solche Vernetzer sind Ethylenglykol, Diethylenglykol, Triethylenglykol, Tetraethylenglykol, Polyethylenglykol, Glycerin, Polyglycerin, Propylenglykol, Diethanolamin, Triethanolamin, Polypropylenglykol, Blockcopolymerisate aus Ethylenoxid und Propylenoxid, Sorbitanfettsäureester, ethoxylierte Sorbitanfettsäureester, Trimethylolpropan, Pentaerythrit, Polyvinylalkohol, Sorbit, Polyglycidylether wie Ethylenglykoldiglycidylether, Polyethylenglykoldiglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanol-tris[3-(1-aziridinyl) propionat], 1,6-Hexamethylendiethylenharnstoff, Diphenylmethan-bis-4,4'-N,N'-diethylenharnstoff, Halogenepoxyverbindungen wie Epichlorhydrin und α-Methylfluorhydrin, Polyisocyanate wie 2,4-Toluylendiisocyanat und Hexamethylendiisocyanat, Alkylencarbonate wie 1,3-Dioxolan-2-on und 4- Methyl-1,3-dioxolan-2-on, polyquaternäre Amine wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Weitere geeignete Vernetzer sind polyvalente Metallionen, die in der Lage sind, ionische Vernetzungen auszubilden. Beispiele für solche Vernetzer sind Magnesium-, Calcium-, Barium- und Aluminiumionen. Diese Vernetzer werden beispielsweise als Hydroxide, Carbonate oder Hydrogencarbonate der wäßrigen polymerisierbaren Lösung zugesetzt.

Weitere geeignete Vernetzer sind multifunktionelle Basen, die ebenfalls in der Lage sind, ionische Vernetzungen auszubilden, beispielsweise Polyamine oder deren quaternierte Salze. Beispiele für Polyamine sind Ethylendiamin, Diethylentriamin, Triethylentetramin, Tetraethylenpentamin, Pentaethylenhexamin und Polyethylenimine sowie Polyvinylamine mit Molmassen von jeweils bis zu 4000000.

In einer bevorzugten Ausführungsform der Erfindung werden zwei unterschiedliche Vernetzer eingesetzt, von denen der eine wasserlöslich und der andere wasserunlöslich ist. Der hydrophile Vernetzer, der in der wäßrigen Phase der Reaktionsmischung löslich ist, bewirkt in konventioneller Weise eine relativ gleichmäßige Vernetzung des entstehenden Polymeren, wie es bei der Herstellung eines Superabsorbers üblich ist. Der hydrophobe Vernetzer, der in der polymerisierbaren wäßrigen Mischung unlöslich bzw. darin nur begrenzt löslich ist, reichert sich in der Tensidgrenzschicht zwischen der Gasphase und der polymerisierbaren wäßrigen Phase an. Dadurch wird bei der nachfolgenden Polymerisation die Oberfläche des Schaums stärker vernetzt als der innere Teil des Superabsorberhydrogels. Man erhält dadurch einen Kern-Schale-Aufbau des Schaums unmittelbar bei der Herstellung des Superabsorberschaums. Eine solche starke oberflächliche Vernetzung eines Superabsorberschaums ist bei den bekannten Herstellverfahren des Standes der Technik nur dadurch möglich, daß man einen bereits gebildeten schaumförmigen Superabsorber nachträglich oberflächlich vernetzt. Für diese Nachvernetzung ist bei konventioneller Arbeitsweise ein eigener Prozeßschritt notwendig, der bei dem Verfahren der vorliegenden Erfindung entfallen kann.

Erfindungsgemäße Produkte mit einem Kern-Schale-Aufbau zeigen gegenüber homogen vernetzten Proben hinsichtlich der Aufnahmegeschwindigkeit, Verteilungswirkung und Gelstabilität deutlich verbesserte Eigenschaften. Mit Ausnahme polyvalenter Metallionen sind alle oben beschriebenen wasserunlöslichen Vernetzer, die den unterschiedlichen Gruppen zugeordnet werden können, geeignet, um Schäume mit einem Kern-Schale-Aufbau herzustellen, d.h. Schäume, bei denen die gesamte Oberfläche stärker vernetzt ist als die darunter liegende Schicht, die oben als Kernschicht bezeichnet wurde. Besonders bevorzugte hydrophobe Vernetzer sind Diacrylate oder Dimethacrylate oder Divinylether von Alkandiolen mit 2 bis 25 C-Atomen (verzweigt, linear, mit beliebiger Anordnung der OH-Gruppen) wie z.B. 1,3-Propandiol, 1,4-Butandiol, 1,6-Hexandiol, Neopentylglycol, 1,9-Nonandiol oder 1,2-Dodecandiol, Di-, Trioder Polypropylenglycoldiacrlyate oder -dimethacrylate, Allylacrylat, Allylmethacrylat, Divinylbenzol, Glycidylacrylat oder Glycidylmethacrylat, Allylglycidylether und Bisglycidylether der oben aufgeführten Alkandiole.

Geeignete hydrophile Vernetzer sind beispielsweise N,N'-Methylenbisacrylamid, Polyethylenglycoldiacrylate oder -dimethacrylate mit einem Molekulargewicht $M_N$ von 200 bis 4000, Divinylharnstoff, Triallylamin, Diacrylate oder Dimethacrylate von Additionsprodukten von 2 bis 400 Mol Ethylenoxid an 1 Mol eines Diols oder Polyols oder das Triacrylat eines Additionsprodukts von 20 Mol Ethylenoxid an 1 Mol Glycerin und Vinylether von Additionsprodukten von 2 bis 400 Mol Ethylenoxid an 1 Mol eines Diols oder Polyols.

Die Monomeren der Gruppe (a) sind in der polymerisierbaren wäßrigen Mischung beispielsweise in Mengen von 10 bis 80 und vorzugsweise 20 bis 60 Gew.-% enthalten. Die Monomeren der Gruppe (b) werden nur gegebenenfalls zur Modifizierung der Superabsorberschäume eingesetzt und können in Mengen bis zu 50, vorzugsweise in Mengen bis zu 20 Gew.-% in der polymerisierbaren wäßrigen Mischung enthalten sein. Die Vernetzer (c) sind in der Reaktionsmischung beispielsweise von 0,001 bis 5 und vorzugsweise von 0,01 und 2 Gew.-% vorhanden.

Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wäßrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden.

Als Polymerisationsinitiatoren können sämtliche unter den Polymerisationsbedingungen in Radikale zerfallende Verbindungen eingesetzt werden, z.B. Peroxide, Hydroperoxide, Wasserstoffperoxid, Persulfate, Azoverbindungen und die sogenannten Redoxkatalysatoren. Bevorzugt ist der Einsatz von wasserlöslichen Initiatoren. In manchen Fällen ist es vorteilhaft, Mischungen verschiedener Polymerisationsinitiatoren zu verwenden, z.B. Mischungen aus Wasserstoffperoxid und Natrium- oder Kaliumperoxidisulfat. Mischungen aus Wasserstoffperoxid und Natriumperoxidisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, Di-(2-ethylhexyl)-peroxidicarbonat, Dicyclohexylperoxydicarbonat, Di-(4-tert.-butylcyclohexyl)peroxidicarbonat, Dimyristyl-peroxidicarbonat, Diacetylperoxydicarbonat, Allylperester, Cumylperoxyneodecanoat, tert.-Butylper-3,5,5-tri-methylhexanoat, Acetylcyclohexylsulfonylperoxid, Dilauroylperoxid, Dibenzoylperoxid und tert.-Amylperneodekanoat. Besonders geeignete Polymerisationsinitiatoren sind wasserlösliche Azostarter, z.B. 2,2'-Azobis-(2-amidinopropan)dihydrochlorid, 2,2'-Azobis-(N,N'-dimethylenisobutyramidin)-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z. B. in Mengen von 0,01 bis 5, vorzugsweise 0,1 bis 2,0 Gew.%, bezogen auf die zu polymerisierenden Monomeren.

Als Initiatoren kommen außerdem Redoxkatalysatoren in Betracht. Die Redoxkatalysatoren enthalten als oxidierende Komponente mindestens eine der oben angegebenen Perverbindungen und als reduzierende Komponente beispielsweise Ascorbinsäure, Glukose, Sorbose, Ammonium- oder Alkalimetall-hydrogensulfit, -sulfit, -thiosulfat, -hyposulfit, -pyrosulfit oder -sulfid, Metallsalze, wie Eisen-II-ionen oder Silberionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise $3.10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte $\alpha$-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanthon-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis (p-azidobenzyliden)cyclohexanon und 2,6-Bis(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Die polymerisierbaren wäßrigen Mischungen enthalten als Komponente (e) 0,1 bis 20 Gew.-% mindestens eines Tensids. Die Tenside sind für die Herstellung und die Stabilisierung des Schaums von entscheidender Bedeutung. Man kann anionische, kationische oder nichtionische Tenside oder Tensidmischungen verwenden, die miteinander verträglich sind. Man kann niedermolekulare oder auch polymere Tenside einsetzen, wobei sich Kombinationen unterschiedlicher oder auch gleichartiger Typen von Tensiden als vorteilhaft herausgestellt haben. Nichtionische Tenside sind beispielsweise Additionsprodukte von Alkylenoxiden, insbesondere Ethylenoxid, Propylenoxid und/oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 C-Atome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für nichtionische Tenside sind die Additionsprodukte von 7 Mol Ethylenoxid an 1 Mol Talgfettalkohol, Umsetzungsprodukte von 9 Mol Ethylenoxid mit 1 Mol Talgfettalkohol und Additionsprodukte von 80 Mol Ethylenoxid an 1 Mol Talgfettalkohol. Weitere handelsübliche nichtionische Tenside bestehen aus Umsetzungsprodukten von Oxoalkoholen oder Ziegler-Al-koholen mit 5 bis 12 Mol Ethylenoxid pro Mol Alkohol, insbesondere mit 7 Mol Ethylenoxid. Weitere handelsübliche nichtionische Tenside werden durch Ethoxylierung von Rizinusöl erhalten. Pro Mol Rizinusöl werden beispielsweise 12 bis 80 Mol Ethylenoxid angelagert. Weitere handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines $C_{13}/C_{15}$-Oxoalkohols, oder die Umsetzungsprodukte von 7 bis 8 Mol Ethylenoxid an 1 Mol eines $C_{13}/C_{15}$-Oxoalkohols. Wietere geeignete nichtionische Tenside sind Phenolalkoxylate wie beispielsweise p-tert.-Butylphenol, das mit 9 Mol Ethylenoxid umgesetzt ist, oder Methylether von Umsetzungsprodukten aus 1 Mol eines $C_{12}$- bis $C_{18}$-Alkohols und 7,5 Mol Ethylenoxid.

Die oben beschriebenen nichtionischen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäure oder von Alkylphenolethersulfaten. Produkte der genannten Art sind im Handel erhältlich. Beispielsweise sind das Natriumsalz eines Schwefelsäurehalbesters eines mit 106 Mol Ethylenoxid umgesetzten $C_{13}/C_{15}$-Oxoalkohols, das Triethanolaminsalz von Dodecylbenzolsulfonsäure, das Natriumsalz von Alkylphenolethersulfaten und das Natriumsalz des Schwefelsäurehalbesters eines Umsetzungsprodukts von 106 Mol Ethylenoxid mit 1 Mol Talgfettalkohol handelsübliche anionische Tenside. Weitere geeignete anionische Tenside sind Schwefelsäurehalbester von $C_{13}/C_{15}$-Oxoalkoholen, Paraffinsulfonsäuren wie $C_{15}$-Alkylsulfonat, alkylsubstituierte Benzolsulfonsäuren und alkylsubstituierte Naphthalinsulfonsäuren wie Dodecylbenzolsulfonsäure und Di-n-butylnaphthalinsulfonsäure sowie Fettalkoholphosphate wie $C_{15}/C_{18}$-Fettalkoholphosphat. Die polymerisierbare wäßrige Mischung kann Kombinationen aus einem nichtionischen Tensid und einem anionischen Tensid oder Kombinationen aus nichtionischen Tensiden oder Kombinationen aus anionischen Tensiden enthalten. Auch kationische Tenside sind geeignet. Beispiele hierfür sind die mit Dimethylsulfat quaternierten Umsetzungsprodukte von 6,5 Mol Ethylenoxid mit 1 Mol Oleylamin, Distearyldimethylammoniumchlorid, Lauryltrimethylammoniumchlorid, Cetylpyridiniumbromid und mit Dimethylsulfat quaternierter Stearinsäuretriethanolaminester, der bevorzugt als kationisches Tensid eingesetzt wird.

Der Tensidgehalt der polymerisierbaren wäßrigen Mischung beträgt 0,1 bis 20, vorzugsweise 0,5 bis 10 Gew.-%. In den meisten Fällen weisen die polymerisierbaren wäßrigen Mischungen einen Tensidgehalt von 1,5 bis 6 Gew.-% auf.

Die polymerisierbaren wäßrigen Mischungen können als Komponente (f) gegebenenfalls mindestens einen Lösevermittler enthalten. Hierunter sollen mit Wasser mischbare organische Lösemittel verstanden werden, z.B. Alkohole, Glykole, Polyethylenglykole bzw. davon abgeleitete Monoether, wobei die Monoether keine Doppelbindungen im Molekül enthalten. Geeignete Ether sind Methylglykol, Butylglykol, Butyldiglykol, Methyldiglykol, Butyltriglykol, 3-Ethoxy-1-propanol und Glycerinmonomethylether.

Die polymerisierbaren wäßrigen Mischungen enthalten 0 bis 50 Gew.-% mindestens eines Lösevermittlers. Falls Lösevermittler eingesetzt werden, beträgt ihr Gehalt in der polymerisierbaren wäßrigen Mischung vorzugsweise bis 25 Gew.-%.

Die polymerisierbare wäßrige Mischung kann gegebenenfalls Verdicker, Schaumstabilisatoren, Polymerisationsregler, Füllstoffe und Zellkeimbildner enthalten. Verdicker werden beispielsweise zur Optimierung der Schaumstruktur und zur Verbesserung der Schaumstabilität eingesetzt. Man erreicht damit, daß der Schaum während der Polymerisation nur geringfügig schrumpft. Als Verdickungsmittel kommen alle hierfür bekannten natürlichen und synthetischen Polymeren in Betracht, die die Viskosität eines wäßrigen Systems stark erhöhen. Hierbei kann es sich um wasserquellbare oder wasserlösliche synthetische und natürliche Polymere handeln. Als Verdicker sind auch pulverförmige Superabsorber geeignet.

Eine ausführliche Übersicht über Verdicker findet man beispielsweise in den Veröffentlichungen von R.Y. Lochhead und W.R. Fron, Cosmetics&Toiletries, 108, 95-135 (Mai 1993) und M.T. Clarke, "Rheological Additives" in D. Laba (ed.) "Rheological Properties of Cosmetics and Toiletries", Cosmetic Science and Technology Series, Vol. 13, Marcel Dekker Inc., New York 1993.

Als Verdicker in Betracht kommende wasserquellbare oder wasserlösliche synthetische Polymere sind beispielsweise hochmolekulare Polymerisate der oben unter (a) beschriebenen Säuregruppen enthaltenden monoethylenisch ungesättigten Monomeren. Solche Verdicker sind beispielsweise hochmolekulare Homopolymerisate von Acrylsäure und/oder Methacrylsäure oder geringfügig vernetzte Copolymerisate aus Acrylsäure und/oder Methacrylsäure und einer Verbindung, die mindestens 2 ethylenisch ungesättigte Doppelbindungen enthält, z.B. Butandioldiacrylat. Außerdem eignen sich hochmolekulare Polymerisate von Acrylamid und Methacrylamid oder Copolymerisate aus Acrylsäure und Acrylamid mit Molmassen von mehr als 1 Million. Solche Copolymerisate sind als Verdickungsmittel bekannt. Auch hochmolekulare Polyethylenglykole oder Copolymerisate aus Ethylenglykol und Propylenglykol sowie hochmolekulare Polysaccharide wie Stärke, Guarkernmehl, Johannisbrotkernmehl oder Derivate von Naturstoffen wie Carboxymethylcellulose Hydroxyethylcellulose, Hydroxymethylcellulose, Hydroxypropylcellulose und Cellulose Mischether sind bekannte Verdicker. Eine weitere Gruppe von Verdickern sind wasserunlösliche Produkte, wie feinteiliges Siliciumdioxid, pyrogene Kieselsäuren, Fällungskieselsäuren in hydrophilen oder hydrophoben Modifikationen, Zeolithe, Titandioxid, Cellulosepulver, oder andere von Superabsorbern verschiedene feinteilige Pulver von vernetzten Polymerisaten. Die polymerisierbaren wäßrigen Mischungen können die Verdicker in Mengen bis zu 30 Gew.-% enthalten. Falls solche Verdickungsmittel überhaupt eingesetzt werden, sind sie in Mengen von 0,1, vorzugsweise 0,5 bis 20 Gew.% in der polymerisierbaren wäßrigen Mischung enthalten.

Um die Schaumstruktur zu optimieren, kann man gegebenenfalls Kohlenwasserstoffe mit mindestens 5 C-Atomen im Molekül zu der wäßrigen Reaktionsmischung zusetzen. Geeignete Kohlenwasserstoffe sind beispielsweise Pentan, Hexan, Cyclohexan, Heptan, Octan, Isooctan, Decan und Dodecan. Die in Betracht kommenden aliphatischen Kohlenwasserstoffe können geradkettig, verzweigt oder zyklisch sein und haben eine Siedetemperatur, die oberhalb der Temperatur der wäßrigen Mischung während des Schäumens liegt. Die aliphatischen Kohlenwasserstoffe erhöhen die Standzeit der noch nicht polymerisierten geschäumten wäßrigen Reaktionsmischung. Dadurch wird das Handling der noch nicht polymerisierten Schäume erleichtert und die Prozeßsicherheit erhöht. Die Kohlenwasserstoffe werden in Mengen von 0 bis 10 Gew.%, bezogen auf die polymerisierbare wäßrige Mischung eingesetzt. Im Fall ihres Einsatzes betragen die bevorzugt in der wäßrigen Mischung vorliegenden Mengen 0,1 bis 5 Gew.-%.

Um die Eigenschaften der Superabsorber zu variieren, beispielsweise die Aufnahmegeschwindigkeit und die Aufnahmekapazität von Wasser, kann es von Vorteil sein, der wäßrigen Reaktionsmischung einen Polymerisationsregler oder eine Mischung mehrerer Polymerisationsregler zuzusetzen. Geeignete Polymerisationsregler sind beispielsweise Ameisensäure, Thioverbindungen wie 2-Mercaptoethanol, Mercaptopropanol, Mercaptobutanol, Dodecylmercaptan, Thioglykolsäure oder Amine wie Ethanolamin, Diethanolamin, Triethanolamin, Triethylamin, Morpholin oder Piperidin. Die Mengen an Polymerisationsregler können bis zu 10 Gew.-%, bezogen auf die eingesetzten Monomeren, betragen. Falls Polymerisationsregler eingesetzt werden, verwendet man vorzugsweise 0,1 bis 5 Gew.-%, bezogen auf die Monomeren.

Die unter (g) angegebenen fakultativ zu verwendenden Bestandteile können einzeln, oder in Mischung bei der Herstellung der erfindungsgemäßen Polymerisate eingesetzt werden. Man kann jedoch auch in Abwesenheit von Verdickern, Schaumstabilisatoren, Füllstoffe, Zellkeimbildner und Polymerisationsreglern arbeiten.

Bei der erfindungsgemäßen Herstellung von wasserabsorbierenden, schaumförmigen, vernetzten Polymerisaten wird in einer ersten Verfahrensstufe die oben beschriebene polymerisierbare wäßrige Mischung geschäumt. Zu diesem Zweck wird in der wäßrigen Monomerphase ein gegenüber Radikalen inertes Gas in Form von feinen Blasen in der Art dispergiert, daß sich ein Schaum bildet. Das Eintragen von Gasblasen in die Monomermischung gelingt beispielsweise mit Hilfe von Schlag-, Schüttel-, Rühr- oder Peitschvorrichtungen. Ferner ist es möglich solche Schäume dadurch herzustellen, daß Gase aus einer flüssigkeitsbedeckten Öffnung ausströmen oder durch das Ausnutzen von Turbulenzerscheinungen in Strömungen. Schließlich kann auch die Ausbildung von Lamellen an Drähten oder Sieben für diesen Zweck genutzt werden. Diese unterschiedlichen Methoden können auch gegebenenfalls miteinander kombiniert werden. Als gegenüber Radikalen inerte Gase eignen sich beispielsweise Stickstoff, Kohlendioxid, Helium, Neon und Argon. Vorzugsweise verwendet man Stickstoff.

Die Herstellung des Schaums erfolgt erfindungsgemäß getrennt von der Polymerisation. Die polymerisierbare wäßrige Mischung kann beispielsweise in technischen Apparaturen geschäumt werden, die für die Herstellung von Harnstoff-Formaldehyd-Schäumen bekannt sind, vgl. Frisch und Saunders, Polymeric Foams Part II, S. 679 ff (1973). Das Schäumen der polymerisierbaren wäßrigen Mischung kann im Labor im einfachsten Fall in einer konventionellen Küchenmaschine erfolgen, die mit Schneebesen bestückt ist. Die Schlagschaumerzeugung wird vorzugsweise in einer Inertgasatmosphäre durchgeführt. Als Inertgase sind beispielsweise Stickstoff, Edelgase oder Kohlendioxid verwendbar. Zur Herstellung des Schaums werden alle Komponenten der Reaktionsmischung vereinigt. Zweckmäßigerweise geht man dabei so vor, daß zunächst alle wasserlöslichen Komponenten in Wasser gelöst werden und daß man erst danach die wasserunlöslichen Stoffe zusetzt. Je nach verwendetem Verfahren der Schlagschaumerzeugung und in Abhängigkeit von dem in der polymerisierbaren wäßrigen Mischung enthaltenen Initiator kann es auch von Vorteil sein, den Initiator erst am Ende des Aufschlagprozesses der Mischung zuzusetzen. Die Konsistenz der Schlagschäume kann in einem weiteren Bereich variiert werden. So ist es möglich, leichte fließfähige Schlagschäume oder aber steife, schnittfeste Schäume herzustellen. Ebenso kann man die mittlere Größe der Gasblasen, ihre Größenverteilung und ihre Anordnung in der Flüssigkeitsmatrix durch die Auswahl der Tenside, der Lösevermittler, Verdicker und Schaumstabilisatoren, Zellkeimbildner, der Temperatur und der Aufschlagtechnik in einem weiten Bereich variieren, so daß man in einfacher Weise Dichte, Offenzelligkeit oder Wandstärke des Matrixmaterials einstellen kann. Die Temperaturen der polymerisierbaren wäßrigen Mischung liegen während des Schäumvorgangs in dem Bereich von -10 bis 100, vorzugsweise 0 bis +50°C. In jedem Falle werden bei der Schaumerzeugung Temperaturen angewandt, die unterhalb des Siedepunkts von Bestandteilen der polymerisierbaren wäßrigen Mischung liegen. Die Schaumerzeugung kann auch unter erhöhtem Druck erfolgen, z.B. bei 1,5 bis 25 bar. Bevorzugt wird jedoch unter Atmosphärendruck gearbeitet.

Gegenüber den bisher bekannten Verfahren zur Herstellung von schaumförmigen Superabsorbern ist ein wesentlicher Vorteil der erfindungsgemäßen Herstellung solcher Schäume darin zu sehen, daß man in der ersten Verfahrensstufe des erfindungsgemäßen Verfahrens geschäumte, polymerisierbare wäßrige Mischungen erhält, die über einen längeren Zeitraum, z.B. bis zu 6 Stunden stabil sind, so daß sie beispielsweise problemlos gehandhabt werden können. Die noch nicht polymerisierten schaumförmigen Mischungen können beispielsweise für die nachfolgende Polymerisation in eine geeignete Form gebracht werden, um die für eine bestimmte Anwendung gewünschten Formkörper herzustellen. Der bei der Formgebung der geschäumten polymerisierbaren wäßrigen Mischung möglicherweise anfallende Abfallschaum kann ohne weiteres in den Prozeß zurückgeführt werden. Das geschäumte polymerisierbare Material kann beispielsweise in der gewünschten Stärke auf ein temporäres Trägermaterial, das vorteilhafterweise mit einer Antihaftbeschichtung ausgestattet ist, aufgetragen werden. Man kann beispielsweise den Schaum auf eine Unterlage aufrakeln. Eine andere Möglichkeit besteht darin, die polymerisierbare schaumförmige wäßrige Mischung in Formen einzufüllen, die ebenfalls antihaftbeschichtet sind und den Schaum darin auszupolymerisieren.

Da die geschäumte polymerisierbare wäßrige Mischung eine lange Standzeit aufweist, eignet sich diese Mischung auch für die Herstellung von Verbundmaterialien. So kann beispielsweise der nach der Schlagschaumerzeugung hergestellte polymerisierbare Schaum auf ein permanentes Trägermaterial aufgebracht werden, z.B. Folien aus Polymeren (z.B. Folien aus Polyethylen, Polypropylen oder Polyamid) oder Metallen, Vliesen, Fluff, Tissues, Gewebe, natürliche oder synthetische Fasern, oder auf andere Schäume. Bei der Herstellung von Verbundmaterialien kann es unter Umständen auch vorteilhaft sein, den polymerisierbaren Schaum in Gestalt von bestimmten Strukturen oder in unterschiedlicher Schichtdicke auf ein Trägermaterial aufzubringen. Es ist jedoch auch möglich, den polymerisierbaren Schaum auf Fluff-Schichten aufzutragen und sie so zu imprägnieren, daß der Fluff nach der Polymerisation integraler Bestandteil des Schaums ist. Die in der ersten Verfahrensstufe erhältliche geschäumte polymerisierbare wäßrige Mischung kann auch zu großen Blöcken geformt und polymerisiert werden. Die Blöcke können nach der Polymerisation zu kleineren Formkörpern geschnitten oder gesägt werden. Man kann auch sandwichartige Strukturen herstellen, indem man eine geschäumte polymerisierbare wäßrige Mischung auf eine Unterlage aufträgt, die schaumförmige Schicht mit einer Folie, Vliesen, Tissues, Geweben, Fasern oder anderen Schäumen gegebenenfalls aus einem anderen Material als die zunächst verwendete Unterlage abdeckt und wiederum Schaum aufträgt und gegebenenfalls mit einer weiteren Folie, Vliesen, Tissues, Geweben, Fasern oder anderen Schäumen abdeckt. Der Verbund wird dann in der zweiten Verfahrensstufe der Polymerisation unterworfen. Man kann jedoch auch sandwichartige Strukturen mit weiteren Schaumschichten herstellen.

In der zweiten Stufe des Verfahrens zur Herstellung der erfindungsgemäßen Superabsorber erfolgt die Polymerisation der geschäumten polymerisierbaren wäßrigen Mischung. Die Polymerisation kann je nach verwendetem Initiator durch Temperaturerhöhung, durch Lichteinwirkung, durch Bestrahlen mit Elektronenstrahlen oder auch durch Temperaturerhöhung und Lichteinwirkung erfolgen. Um die Temperatur der geschäumten polymerisierbaren wäßrigen Mischung zu erhöhen, kann man alle in der Technik üblichen Verfahren anwenden, beispielsweise den Schaum mit heizbaren Platten in Kontakt bringen, Einwirkung von Infrarotbestrahlung auf den polymerisierbaren Schaum oder Beheizen mit Hilfe von Mikrowellen. Falls dickere Schichten eines Schaumstoffs hergestellt werden sollen, z.B. Schäume mit Dicken von mehreren Zentimetern, ist die Erwärmung des polymerisierbaren geschäumten Materials mit Hilfe einer Mikrowelle besonders vorteilhaft, weil auf diesem Wege eine relativ gleichmäßige Erwärmung erreicht werden kann.

Die Polymerisation erfolgt beispielsweise bei Temperaturen von 20 bis 180, vorzugsweise in dem Bereich von 20 bis 100°C.

Bei Initiierung der Polymerisation durch Lichteinwirkung auf das geschäumte polymerisierbare Material kann man alle konventionellen Belichtersysteme anwenden, sofern ihr Emissionsspektrum an den eingesetzten Photoinitiator adaptiert ist. Bei einem Start der Polymerisation durch Belichten wird vorteilhaft eine Kombination eines Photoinitiators und eines thermischen Initiators oder/ und aber ein Photoinitiator eingesetzt, der auch als thermischer Initiator wirken kann, z.B. Azoinitiatoren. Da sich der Schaum während der Polymerisation durch die hohe Polymerisationswärme stark erwärmt, wird auf diese Weise ein besonders schneller und effektiver Ablauf der Polymerisationsreaktion erreicht. Bei der Initiierung durch Lichteinwirkung liegt die Polymerisationstemperatur in dem Bereich von 0 bis 150, vorzugsweise 10 bis 100°C.

Ein wesentlicher Vorteil des erfindungsgemäßen Verfahrens ist darin zu sehen, daß die Polymerisation unter weitgehendem Erhalt der Struktur der geschäumten polymerisierbaren wäßrigen Mischung abläuft, d.h. der polymerisierbare Schaum verändert während der Polymerisation sein Volumen nur unwesentlich. Die Polymerisationsreaktion wird durch die Starttemperatur, die Initiierungstechnik oder die Wärmeabfuhr beeinflußt. Die Polymerisationstemperatur wird vorzugsweise dahingehend kontrolliert, daß ein Sieden der polymerisierbaren wäßrigen Mischung vermieden wird. Mit fortschreitender Polymerisation tritt eine Verfestigung des Schaums infolge zunehmender Gelbildung ein. Nach Beendigung der Polymerisation liegt ein schaumförmiges Hydrogel vor, daß einen Wassergehalt von 30 bis 80 Gew.-% hat. Der Schaum hat zumindest teilweise eine offenzellige Struktur. Für die Anwendung des Schaums als Superabsorber ist eine Restfeuchte von 1 bis 45, vorzugsweise 15 bis 35 Gew.% wünschenswert. Das bei der Polymerisation anfallende schaumförmige Hydrogel wird daher meistens getrocknet. Um einen flexiblen Schaum zu erhalten, muß der Schaum eine gewisse Restfeuchte aufweisen. Der Wassergehalt hängt stark von der Dichte des erzeugten Schaums ab. Je höher die Dichte ist, desto mehr Restfeuchte ist einzustellen. Daher kann eine Obergrenze von 35 bis 45 Gew.-% Wasser durchaus sinnvoll sein. Wird ein Ansatz mit sehr hohem Festgehalt polymerisiert, der einen Schaum mit einer sehr hohen Dichte ergibt, kann es sogar notwendig sein, nach der Polymerisation den Schaum weiter anzufeuchten, um die notwendige Flexibilität zu erhalten.

Der Schaum kann mit Hilfe aller konventionellen Techniken getrocknet werden, beispielsweise durch Erhitzen mit einem heißen Gasstrom, durch Anlegen von Vakuum, durch Infrarotbestrahlung oder durch Erhitzen mit Mikrowellenstrahlung. Die Mikrowellenstrahlung erweist sich auch hier wiederum beim Trocknen von großvolumigen Formkörpern als vorteilhaft.

Nach dem erfindungsgemäßen Verfahren erhält man einen überwiegend offenzelligen Superabsorberschaum, der relativ hart und spröde ist. Für viele Anwendungen werden jedoch Schäume verlangt, die flexibel sind. Der zunächst erhaltene relativ harte und spröde Schaum kann jedoch flexibilisiert werden. Dies kann mit Hilfe von externen Weichmachern oder durch eine interne Flexibilisierung geschehen.

Externe Weichmacher sind Komponenten, die zusätzlich zu den gelbildenden Komponenten entweder der Reaktionsmischung vor dem Verschäumen zugesetzt werden, oder die nachträglich auf den Schaum aufgetragen werden. Als Weichmacher kommen beispielsweise hydrophile und hygroskopische Substanzen in Betracht. Eine externe Flexibilisierung wird in erster Linie durch das gezielte Einstellen eines bestimmten Restwassergehalts erreicht.

Weiterhin kann die Flexibilisierung durch den Einsatz von beispielsweise Polyolen wie Glycerin, Polyalkylenglykolen wie Polyethylenglykolen oder Polypropylenglykolen, oder kationischen Tensiden verbessert werden. Geeignete kationische Tenside sind beispielsweise mit Dimethylsulfat quaternierte Umsetzungsprodukte von 1 Mol Oleylamin mit 5 bis 10 Mol Ethylenoxid, Distearyldimethylammoniumchlorid, Lauryltrimethylammoniumchlorid, Cetylpyridiniumbromid und Ethanolaminester langkettiger Fettsäuren wie Stearinsäurediethanolaminester, Stearinsäuremonoethanolaminester und Stearinsäuretriethanolaminester, der bevorzugt als externer Weichmacher eingesetzt wird.

Unter interner Flexibilisierung des Schaums wird der Einsatz von weichmachenden Komponenten verstanden, die in die Gelstruktur eingebaut werden. Hierbei kann es sich um Substanzen handeln, die selbst ungesättigte Gruppen tragen und bei der Polymerisation als Monomere (b) in der polymerisierbaren wäßrigen Mischung vorliegen und mit in die Gelstruktur eingebaut werden oder die mit dem gelbildenden Material reagieren. Der interne Weichmacher soll eine Erniedrigung der Glastemperatur des Polymeren bewirken, das den Superabsorber darstellt. Als interne Weichmacher sind beispielsweise Olefine, Ester aus ethylenisch ungesättigten $C_3$-bis $C_5$-Carbonsäuren und einwertigen $C_2$ bis $C_{30}$-Alkoholen oder Polyethylenglykol- oder Polypropylenglykolmonoester von monoethylenisch ungesättigten $C_3$- bis $C_5$-Carbonsäuren geeignet. Zur internen Flexibilisierung eignen sich diejenigen Monomeren (b), die die Glastemperatur der entstehenden Copolymerisate mit den Monomeren (a) herabsetzen, z.B. Vinylester von mindestens 4 C-Atome enthaltenden gesättigten Carbonsäuren, Alkylvinylether mit mindestens 2 C-Atomen in der Alkylgruppe, Vinyllactame und alkylsubstituierte Styrole wie Ethylstyrol.

Wie oben bereits angegeben wurde, kann eine inhomogene Vernetzungsdichte bei den erfindungsgemäßen Superabsorberschäumen bereits während der Herstellung erzeugt werden. Dies ist besonders vorteilhaft, wenn man als Monomere der oben beschriebenen Komponenten

   (a) Acrylsäure, Methacrylsäure, Vinylsulfonsäure, Acrylamidopropansulfonsäure oder deren Mischungen und

   (c) eine Mischung aus mindestens einem wasserlöslichen und mindestens einem wasserunlöslichen Vernetzer einsetzt.

Dennoch kann es wünschenswert sein, den Vernetzungsgrad des Schaumes nachträglich zu verändern. Um dieses Ziel zu erreichen, kann man beispielsweise während der Polymerisation durch den Zusatz geeigneter Monomere in das Gel latente Vernetzungsstellen einbauen, die unter den Bedingungen der Schaumherstellung nicht zu Vernetzungsreaktionen führen, jedoch unter speziellen Bedingungen, die nachträglich angewandt werden können, z.B. durch stark erhöhte Temperatur, in der Lage sind, weitere Vernetzungspunkte in der Gelstruktur zu bilden. Als Beispiele für solche Monomere kann der Einbau von Hydroxygruppen enthaltenden Verbindungen dienen, die bei höherer Temperatur, d.h. bei Temperaturen oberhalb von 150°C in der Lage sind, mit den Carboxylgruppen in der Schaumstruktur zu reagieren. Geeignete Verbindungen, die latente Vernetzungsstellen aufweisen, sind beispielsweise Hydroxyethylacrylat, Hydroxypropylacrylat, Hydroxybutylacrylat, Monoacrylsäureester des Glycerins, Monoacrylate oder Monomethacrylate von Polyethylenglykolen mit mindestens 2 Ethylenglykoleinheiten, Monoacrylate oder Monomethacrylate von Polypropylenglykolen mit mindestens 2 Propylenglykoleinheiten und Monomethacrylate von mehrwertigen Alkoholen, z.B. Hydroxybutylmethacrylat, Hydroxypropylmethacrylat, Hydroxyethylmethacrylat oder Glycerinmonomethacrylat.

Als weitere Möglichkeit einer homogenen Nachvernetzung bietet sich der nachträgliche Zusatz von Vernetzungsreagenzien an, d.h. Verbindungen, die mindestens zwei reaktive Gruppen aufweisen, die unter geeigneten Bedingungen in der Lage sind, z.B. beim Erhitzen auf Temperaturen oberhalb von 70°C, mit den Säuregruppen des schaumförmigen Hydrogel zu reagieren. In diesem Falle ist es auch möglich, gesteuert über die Eindringtiefe des Vernetzers, eine Modifikation der inhomogenen Vernetzungsdichte zu erreichen. Geeignete Vernetzer bilden mit den Carboxylgruppen der Polymermatrix kovalente oder ionische Bindungen. Geeignete Vernetzungsmittel sind Verbindungen, die mindestens zwei funktionelle Gruppen der gleichen oder unterschiedlicher Art aufweisen, z.B. Hydroxy-, Amino-, quaternäre Ammonium-, Isocyanato-, Epoxi-, Aziridino-, Ester- oder Amidgruppen. Bevorzugte Nachvernetzungsmittel sind Polyalkohole wie Glycerin oder Bisepoxide. Das Auftragen der Vernetzungsmittel auf das geschäumte Material kann beispielsweise durch Sprühen, Tauchen oder durch Gasphasenabscheidung erfolgen.

Die erfindungsgemäßen Superabsorberschäume haben beispielsweise eine Dichte von $10^{-3}$ bis 0,9, vorzugsweise 0,05 bis 0,7 g/cm$^3$. Die Dichte von Superabsorberschäumen wird gravimetrisch bestimmt. Aus einer gleichmäßigen Schaumschicht mit einer definierten Dicke zwischen 3 und 5 mm schneidet man beispielsweise mit einem scharfen Messer Quadrate mit einer Seitenlänge von 5 cm aus. Diese Proben werden gewogen und das erhaltene Gewicht durch das aus den Maßen errechnete Volumen dividiert.

Um die aus dem schaumförmigen Superabsorber extrahierbaren Anteile zu bestimmen, wird eine getrocknete und gemahlene Schaumprobe in einer 0,9 gew.-%igen Kochsalzlösung dispergiert und die Dispersion 1 Stunde gerührt. Danach filtriert man das schaumförmige Material ab und bestimmt die Menge des extrahierten Anteils im Filtrat titrimetrisch.

Die Aufnahmekapazität des schaumförmigen Superabsorbers an Wasser pro Gramm Superabsorber wird an Schaumstücken bestimmt, die eine Dicke von 3 mm haben und jeweils 1 g wiegen. Die Prüfung der Retention erfolgt hierbei nach dem sogenannten Teebeuteltest. Als Flüssigkeit dient dabei eine 0,9 %ige Kochsalzlösung. 1 g des schaumförmigen Materials wird in einen Teebeutel gefüllt, der dann verschlossen wird. Der Teebeutel wird danach eine bestimmte Zeit lang in die Flüssigkeit eingetaucht und nach einer Abtropfdauer von 10 Minuten zurückgewogen. Für die Berechnung der Absorptionskapazität muß ein Blindversuch durchgeführt werden, bei dem ein Teebeutel ohne schaumförmigen Superabsorber in die Lösung eingetaucht und das Gewicht des Teebeutels nach der oben angegebenen Abtropfdauer von 10 Minuten bestimmt wird. Die Aufnahmekapazität ergibt sich dann aus folgender Beziehung

$$\text{Aufnahmekapazität} = \frac{\text{Gewicht des Teebeutels mit Superabsorberschaum - Gewicht des Teebeutels im Blindversuch}}{\text{Gewicht des eingewogenen Superabsorberschaums}}$$

Die Retention wird folgendermaßen ermittelt:
Gleiche Vorgehensweise wie oben, nur wird anstelle des Abtropfens der Teebeutel 3 min in einer Zentrifuge mit einer Beschleunigung von 250 g geschleudert.

$$\text{Retention} = \frac{\text{Gewicht des Teebeutels nach dem Schleudern - Gewicht des Teebeutels im Blindversuch}}{\text{Gewicht des eingewogenen Gewichts des Superabsorberschaums}}$$

Die Aufnahmegeschwindigkeit (Absorption Speed, im folgenden mit AS bezeichnet) wurde dadurch ermittelt, daß man aus gleichmäßig 3 mm dicken Schaumschichten rechteckige Proben mit einem Gewicht von 1 g mit Hilfe eines scharfen Messers ausschnitt. Diese Proben wurden in einer Petrischale mit 20 g synthetischem Urin übergossen. Mit Hilfe einer Stoppuhr wurde die Zeit ermittelt, die der Schaumstoff benötigte, um den synthetischen Urin vollständig aufzunehmen. Die Aufnahmegeschwindigkeit (AS) in g/g·sec errechnet sich zu:

$$AS = 20 \text{ g}/[1 \text{ g}^* \text{ gemessene Zeit (in sec)}]$$

Ferner wird bei diesem Test die Gleichmäßigkeit der Flüssigkeitsaufnahme nach einer 6-stufigen Notenskala bewertet. Die Noten 1-6 haben folgende Bedeutung:

1:   Der Schaum quillt von Anfang an homogen.
2:   Der Schaum quillt nach wenigen Sekunden homogen.
3:   Der Schaum quillt nach 30 sec homogen.
4:   Der Schaum quillt die ganze Zeit inhomogen, aber nur ein kleiner Teil ist davon betroffen.
5:   Der Schaum quillt die ganze Zeit inhomogen, aber ein wesentlicher Teil ist davon betroffen.
6:   Der Schaum quillt die ganze Zeit nur an der Oberfläche.

Rezeptur für synthetischen Urin:

in 1 1 destilliertem Wasser werden die folgenden Salze gelöst:

| | |
|---|---|
| 2,00 g | KCl |
| 2,00 g | $Na_2SO_4$ |
| 0,85 g | $NH_4H_2PO_4$ |
| 0,15 g | $(NH_4)_2HPO_4$ |
| 0,19 g | $CaCl_2$ |
| 0,23 g | $MgCl_2$ |

Die eingesetzten Salze müssen wasserfrei sein.

Stabilität des Schaums im gequollenen Zustand

Anhand der in obigem Test erhaltenen Proben wurde die Stabilität des ausgequollenen Materials nach einer 4-stufigen Notenskala bewertet. Die Noten 1-4 bedeuten dabei:

1:   Der Schaum kann unversehrt aus der Petrischale genommen werden und kann um 180° gebogen werden, ohne daß er reißt.

2: Der Schaum kann unversehrt aus der Petrischale genommen werden.

3: Der Schaum reißt beim herausnehmen aus der Petrischale.

4: Der Schaum zerfällt zu einem unzusammenhängenden Gelhaufen.

Die oben beschriebenen wasserabsorbierenden, schaumförmigen, vernetzten Polymerisate können für sämtliche Zwecke verwendet werden, für die die in der Literatur beschriebenen schaumförmigen Superabsorber eingesetzt werden. Sie werden z.B. in Sanitärartikeln, die zur Adsorption von Körperflüssigkeiten eingesetzt werden und in Verbandmaterial zur Abdeckung von Wunden verwendet. Sie eignen sich beispielsweise als wasserabsorbierender Bestandteil in Windeln, Damenbinden und Inkontinenzartikeln. Sie können in Form von Verbundmaterialien eingesetzt werden. Schaumförmige Superabsorber können außerdem als Dichtungsmaterial, als Bodenverbesserungsmittel, als Bodenersatzstoff und als Verpackungsmaterial verwendet werden. Spezielle Ausgestaltungen von Gegenständen, die schaumförmige Superabsorber enthalten, werden beispielsweise ausführlich in der WO-A-94/22502 beschrieben. Die schaumförmigen Superabsorber eignen sich außerdem zur Entwässerung von Schlämmen, zum Eindicken wäßriger Lacke, z.B. für die Entsorgung von Restmengen nicht verbrauchter wäßriger Lacke oder Farben, indem man z.B. pulverförmige schaumförmige Superabsorber zu wäßrigen Lackresten zugibt, bis eine Verfestigung eintritt. Die schaumförmigen, wasserabsorbierenden, vernetzten Polymerisate können außerdem zur Entwässerung von wasserhaltigen Ölen verwendet werden. Sie können beispielsweise in Form eines Pulvers mit einem mittleren Teilchendurchmesser von 150 µm bis 5 mm bei den oben beschriebenen Anwendungen eingesetzt werden.

Die oben beschriebenen Schäume können aufgrund ihrer Eigenschaften verschiedene Funktionen in Hygieneartikeln bei der Speicherung von Körperflüssigkeiten erfüllen:

- Akquisition
- Distribution und/oder
- Speicherung

Die Speicherung von Körperflüssigkeiten wird von den Schäumen vollständig übernommen, während für die Funktionen Akquisition und Distribution gegebenenfalls weitere Bestandteile wie high loft-Nonwovens, Polypropylen-Vliese, Polyester-Vliese oder chemisch modifizierte Zellstoffe unterstützend als Schicht auf den Schäumen Verwendung finden können.

Die Prozentangaben in den Beispielen bedeuten Gewichtsprozent, sofern aus dem Zusammenhang nichts anderes hervorgeht.

Beispiele

Beispiel 1

In einem Becherglas werden mit Hilfe eines Magnetrührers die folgenden Komponenten vermischt:

| | |
|---|---|
| 224,20 g | einer 37,3 %igen Natriumacrylatlösung in Wasser |
| 21,36 g | Acrylsäure |
| 1,05 g | Triacrylsäureester eines mit 20 Ethylenoxid veretherten Glycerins |
| 3,15 g | Additionsprodukt von 80 Mol Ethylenoxid an 1 Mol Talgfettalkohol |
| 0,53 g | 1,4-Butandioldiacrylat |
| 4,30 g | Pentan |
| 49,68 g | Wasser |

Die erhaltene homogene Mischung wird in einen 2 l-Kolben eingefüllt, in den von unten her Argon eingeleitet wird. In den Kolben sind 2 Schneebesen eingesetzt, die mit jeweils einem Rührer der Firma Janke & Kunkel Typ RW 20 DZM verbunden sind. Der Argonstrom wird so eingestellt, daß er mit einer Geschwindigkeit von 2,5 l/h durch die Reaktionsmischung perlt. Die beiden Rührer werden zunächst auf eine Drehzahl von 60 UpM eingestellt. Zu der Reaktionsmischung werden 45,00 g feingemahlener Superabsorber (Partikelgröße < 100 µm) gegeben und homogen untergemischt. Die freie Öffnung des Kolbens wird fast vollständig mit Parafilm abgedichtet und die Rührerdrehzahl auf 1000 UpM erhöht. Die Mischung wird bei dieser Drehzahl für 20 min bei Raumtemperatur aufgeschlagen. 5 Minuten vor Ende des Aufschlagprozesses werden 11,9 g einer 3 %igen Lösung von 2,2'-Azobis(2-amidinopropan)dihydrochlorid in den Kolben gegeben. Nach Ende der Aufschlagperiode wird ein feinteiliger, gut fließfähiger Schlagschaum erhalten.

Der Schaum wird in einer Schichtdicke von 3 mm in eine Kiste aus Polypropylen (Maße: Breite 20 cm, Tiefe 20 cm, Höhe 15 cm) gefüllt, die zuvor mit Argon gespült wurde. In dieser Kiste wird der Schaum für 60 sec mit einem Belichter bestrahlt. Erhalten wird eine gleichmäßig 3 mm dicke leicht flexible Schaumschicht, die sich leicht aus der Kiste herausnehmen läßt. Sie wird in einem Vakuumtrockenschrank bei 70°C und einem Vakuum von 20 mbar auf einen Restwassergehalt von 25 % getrocknet. Zur Äquilibrierung wird die getrocknete Schaumschicht über Nacht in einer festverschlossenen Tüte aus Polyethylen gelagert. Danach ist die erhaltene Schaumschicht nach wie vor weich und flexibel. Für Testzwecke wird ein kleiner Teil der Probe im Vakuum vollständig getrocknet.

Eigenschaften des Superabsorberschaums:

| | |
|---|---|
| Dichte | 650 g/l |
| Extrahierbare Anteile | 5,7 % |
| Aufnahme | 23,5 g/g |
| Retention | 10,6 g/g |
| AS | 1,1 g/g·sec |
| Gleichmäßigkeit der Absorption | Note 1 |
| Stabilität im gequollenen Zustand | Note 1 |

Beispiel 2

| Einsatzstoffe | 400,00 g | Na-Acrylatlösung (37,3 %ig) |
|---|---|---|
| | 38,10 g | Acrylsäure |
| | 2,00 g | Trimethylolpropantriacrylat (TMPTA) |
| | 21,22 g | 2,2'-Azobis-(N,N' -dimethyleneisobutyramidin)dihydrochlorid (3 %ige wäßrige Lösung) |
| | 10,00 g | Na-Salz eines $C_{13}/C_{15}$-Oxoalkoholschwefelsaurehalbesters |

In einem Schraubdeckelgefäß wird zunächst der Vernetzer (TMPTA) in der Acrylsäure gelöst. Unter Rühren werden das Na-Acrylat sowie der Emulgator zugegeben. Der Ansatz wird mittels Magnetrührer mindestens 4 Stunden gerührt. In einer handelsüblichen Bosch-Küchenmaschine mit Abdeckung wird der Ansatz bei höchster Rührstufe zu einem Schaum aufgeschlagen. Während dieses Vorgangs wird der Luftraum über dem Schaum ständig mit Kohlendioxid gefüllt. Nach 15 Minuten wird die Starterlösung zugegeben und weitere 5 Minuten geschlagen. Es entsteht ein feiner, gut fließfähiger Schlagschaum von ca. 3 Liter Schaumvolumen.

Der Schaum wird in eine 15,5x19,0x18,0 cm große Form aus Polypropylen umgefüllt und 10 Minuten in der Mikrowelle polymerisiert.

Es wird ein 7 cm dicker Schaumblock erhalten.

Eigenschaften des Superabsorberschaums:

| | |
|---|---|
| Dichte | 115,3 g/l |
| Extrahierbare Anteile | 10,4 % |
| Aufnahme | 57,4 g/g |
| Retention | 32,9 g/g |
| AS | 0.37 g/g·sec |

Beispiel 3

| Einsatzstoffe | 400,00 g | Na-Acrylatlösung (37,3 %ig) |
|---|---|---|
| | 38,10 g | Acrylsäure |
| | 2,00 g | Trimethylolpropantriacrylat (TMPTA) |
| | 21,22 g | 2,2'-Azobis-(N,N'-dimethylenisobutyramidin)dihydrochlorid (in Form einer 3 %igen wäßrigen Lösung) |

(fortgesetzt)

| | 10,00 g | Na-Salz eines $C_{13}/C_{15}$-Oxoalkoholschwefelsaurehalbesters |
|---|---|---|
| | 10,00 g | einer hydrophilen Fällungskieselsäure mit einer mittleren Teilchengröße von 7 μm |

In einem Schraubdeckelgefäß wird zunächst der Vernetzer (TMPTA) in der Acrylsäure gelöst. Unter Rühren werden das Na-Acrylat, der Emulgator und die Fällungskieselsäure zugegeben. Der Ansatz wird mittels Magnetrührer mindestens 4 Stunden gerührt. In einer handelsüblichen Bosch-Küchenmaschine mit Abdeckung wird der Ansatz bei höchster Rührstufe zu einem Schaum aufgeschlagen. Während dieses Vorgangs wird der Luftraum über dem Schaum ständig mit Kohlendioxid gefüllt. Nach 15 Minuten wird die Starterlösung zugegeben und weitere 5 Minuten geschlagen. Es entsteht ein feiner, steifer Schlagschaum von ca. 3 Liter Schaumvolumen.

Der Schaum wird in eine 15,5*19,0*18,0 cm große Form aus Polypropylen umgefüllt und 10 Minuten in der Mikrowelle polymerisiert. Man erhält einen 8 cm dicken Schaumblock.

Eigenschaften des Superabsorberschaums:

| Dichte | 87,5 g/l |
|---|---|
| Extrahierbare Anteile | 7,4 % |
| Aufnahme | 52,3 g/g |
| Retention | 23,3 g/g |
| AS | 0,12 g/g·sec |

Beispiel 4

| Einsatzstoffe | 400,00 g | Na-Acrylatlösung (37,3 %ig) |
|---|---|---|
| | 38,10 g | Acrylsäure |
| | 2,00 g | Trimethylolpropantriacrylat |
| | 21,22 g | 2,2'-Azobis-(N,N'-dimethylenisobutyramidin)dihydrochlorid (in Form einer 3 %igen wäßrigen Lösung) |
| | 10,00 g | Na-Salz eines $C_{13}/C_{15}$-Oxoalkoholschwefelsäurehalbesters |
| | 5,00 g | einer hydrophilen Fällungskieselsäure mit einer mittleren Teilchengröße von 7 μm |

In einem Schraubdeckelgefäß wird zunächst der Vernetzer (TMPTA) in der Acrylsäure gelöst. Unter Rühren werden das Na-Acrylat, der Emulgator sowie die Fällungskieselsäure zugegeben. In einer handelsüblichen Bosch-Küchenmaschine mit Abdeckung wird der Ansatz bei höchster Rührstufe zu einem Schaum aufgeschlagen. Während dieses Vorgangs wird der Luftraum über dem Schaum ständig mit Kohlendioxid gefüllt. Nach 15 Minuten wird die Starterlösung zugegeben und weitere 5 Minuten geschlagen. Es entsteht ein feiner, steifer Schlagschaum von ca. 2 Liter Schaumvolumen.

Der Schaum wird in eine 15,5x19,0x18,0 cm große Polypropylenform umgefüllt und 10 Minuten in der Mikrowelle polymerisiert. Man erhält einen 7 cm dicken Schaumblock.

Eigenschaften des Schaums:

| Dichte | 99,1 g/l |
|---|---|
| Extrahierbare Anteile | 9,7 % |
| Aufnahme | 61,3 g/g |
| Retention | 31,1 g/g |
| AS | 0,80 g/g·sec |

Beispiel 5

| Einsatzstoffe | 400,00 g | Na-Acrylat (37 %ig) |
|---|---|---|
| | 38,10 g | Acrylsäure |

(fortgesetzt)

| | 2,00 g | Trimethylolpropantriacrylat |
|---|---|---|
| | 0,64 g | 2,2'-Azobis-(N,N'-dimethylenisobutyramidin)dihydrochlorid (in Form einer 3 %igen wäßrigen Lösung) |
| | 10,00 g | Stearinsäuretriethanolaminester mit Dimethylsulfat quarterniert |
| | 2,50 g | Hydroxyethylcellulose (2 %ige Brookfield Viskosität = 6000 mPas) |

In einem Schraubdeckelgefäß wird zunächst der Vernetzer in Acrylsäure gelöst. Unter Rühren werden das Na-Acrylat, der Emulgator sowie die Hydoxyethylcellulose zugegeben. Der Ansatz wird dann noch über Nacht gerührt. In einer handelsüblichen Bosch-Küchenmaschine mit Deckel wird der Ansatz 20 min zu einem Schaum aufgeschlagen. Während dieses Vorgangs wird der Luftraum über dem Schaum ständig mit Kohlendioxid gefüllt. Danach wird die Starterlösung zugegeben und die polymerisierbare Mischung weitere 5 min geschlagen. Es entsteht ein feiner, steifer Schaum mit ca. 2,5 1 Volumen. Der Schaum wird in eine 15,5x19,0x18,0 cm große Form aus Polypropylen gefüllt und im Mikrowellenofen polymerisiert.

Es wird ein 9 cm dicker Schaumblock erhalten.

Eigenschaften des Superabsorberschaumes

| Dichte | 76,4 g/l |
|---|---|
| Extrahierbare Anteile | 10,3 % |
| Wasseraufnahme | 55,3 g/g |
| Retention | 42,1 g/g |
| AS | 1,2 g/g·sec |

Beispiel 6

| Einsatzstoffe | 400,00 g | 37 %ige wäßrige Na-Acrylatlösung |
|---|---|---|
| | 38,10 g | Acrylsäure |
| | 1,87 g | Triacrylsäureester eines mit 20 Mol Ethylenoxid veretherten Glycerins |
| | 0,64 g | 2,2'Azobis-(N,N'-dimethylenisobutyramidin)dihydrochlorid (in Form einer 3 %igen wäßrigen Lösung) |
| | 10,00 g | Stearinsäuretriethanolaminester mit Dimethylsulfat quarterniert |

Nach der im Beispiel 4 angegebenen Vorschrift wird ein Schaum hergestellt. Es entsteht ein feiner, ziemlich steifer Schlagschaum von ca. 3,5 1 Schaumvolumen. Der Schaum wird in eine 15,5x19,0x18,0 cm große Form aus Polypropylen umgefüllt und 10 Minuten in einem Mikrowellenofen polymerisiert. Man erhält einen 8 cm dicken Schaumblock.

Eigenschaften des Superabsorberschaumes

| Dichte | 84,3 g/l |
|---|---|
| Extrahierbare Anteile | 9,8 % |
| Wasseraufnahme | 58,4 g/g |
| Retention | 43,2 g/g |
| AS | 1,1 g/g·sec |

Beispiel 7

| Einsatzstoffe | 400,00 g | 37 %ige wäßrige Na-Acrylatlösung |
|---|---|---|
| | 31,8 g | Acrylsäure |
| | 2,0 g | Trimethylolpropantriacrylat |

(fortgesetzt)

| | 0,64 g | 2,2'-Azobis-(N,N'-dimethylenisobutyramidin)dihydrochlorid (in Form einer 3 %igen wäßrigen Lösung) |
| | 8,00 g | Na-Salz eines $C_{13}/C_{15}$-Oxoalkoholschwefelsäurehalbesters |
| | 2,00 g | $C_{15}$-Alkylsulfonsäure als Na-Salz |

Die oben angegebenen Einsatzstoffe werden nach der im Beispiel 4 angegebenen Vorschrift geschäumt und polymerisiert. Es entsteht zunächst ein feiner, steifer Schaum mit einem Volumen von 3 1. Nach dem Polymerisieren erhält man einen 6 cm dicken Schaumblock.

Eigenschaften des Superabsorberschaumes

| Dichte | 113,0 g/l |
| Extrahierbare Anteile | 9,5 % |
| Wasseraufnahme | 54,7 g/g |
| Retention | 43,2 g/g |
| AS | 1,3 g/g·sec |

**Patentansprüche**

1.  Wasserabsorbierende, überwiegend offenzellige schaumförmige, vernetzte Polymerisate, dadurch gekennzeichnet, daß sie erhältlich sind durch

    (I) Schäumen einer polymerisierbaren wäßrigen Mischung, die

    (a) 10 bis 80 Gew.-% Säuregruppen enthaltende monoethylenisch ungesättigte Monomere, die zu mindestens 50 Mol% neutralisiert sind,
    (b) gegebenenfalls bis zu 50 Gew.-% andere monoethylenisch ungesättigte Monomere,
    (c) 0,001 bis 5 Gew.-% Vernetzer,
    (d) Initiatoren,
    (e) 0,1 bis 20 Gew.-% mindestens eines Tensids,
    (f) gegebenenfalls mindestens einen Lösevermittler und
    (g) gegebenenfalls Verdicker, Schaumstabilisatoren, Polymerisationsregler, Füllstoffe und/oder Zellkeimbilder

    enthält, wobei das Schäumen durch Dispergieren von feinen Blasen eines gegenüber Radikalen inerten Gases erfolgt, und
    (II) Polymerisieren der geschäumten Mischung unter Bildung eines schaumförmigen Hydrogels und Einstellen des Wassergehalts des schaumförmigen Polymerisats auf 1 bis 45 Gew.-%.

2.  Wasserabsorbierende, schaumförmige, vernetzte Polymerisate nach Anspruch 1, dadurch gekennzeichnet, daß man eine polymerisierbare wäßrige Mischung einsetzt, die als

    (a) Säuregruppen enthaltende monoethylenisch ungesättigte Monomere, die zu mindestens 50 Mol% neutralisiert sind,
    (b) $C_2$- bis $C_{25}$-Olefine, Ester aus monoethylenisch ungesättigten $C_3$- bis $C_5$-Carbonsäuren und einwertigen $C_2$-bis $C_{25}$-Alkoholen, Polyethylenglykol- oder Polypropylenglykolmonoester von monoethylenisch ungesättigten $C_3$- bis $C_5$-Carbonsäuren, Vinylester von mindestens 4 C-Atome enthaltenden gesättigten Carbonsäuren, Alkylvinylether mit mindestens 2 C-Atomen in der Alkylgruppe, alkylsubstituierte Styrole, Hydroxyalkylester von monoethylenisch ungesättigten $C_3$- bis $C_5$-Carbonsäuren, N-Vinyllactame, N-Vinylimidazole mit 5 bis 8 C-Atomen sowie deren Salze und Quarternierungsprodukte, basische Ester oder Amide von monoethylenisch ungesättigten $C_3$- bis $C_5$-Carbonsäuren sowie deren Salze und Quarternierungsprodukte.

    enthält.

3.  Wasserabsorbierende, schaumförmige, vernetzte Polymerisate nach Anspruch 1, dadurch gekennzeichnet, daß man eine polymerisierbare, wäßrige Mischung aus

(a) Säuregruppen enthaltenden monoethylenisch ungesättigten Monomeren, die zumindestens 50 Mol% neutralisiert sind, und

(b) eine Mischung aus mindestens einem wasserlöslichen und mindestens einem wasserunlöslichen Vernetzer einsetzt.

4. Wasserabsorbierende, schaumförmige, vernetzte Polymerisate nach Anspruch 1 oder 2, dadurch gekennzeichnet, daß sie 0,1 bis 10 Gew.-% eines externen Weichmachers aus der Gruppe Polyole, Polyalkylenglykole und der kationischen Tenside enthalten.

5. Wasserabsorbierende, schaumförmige, vernetzte Polymerisate nach Anspruch 1, dadurch gekennzeichnet, daß die gesamte Oberfläche nachvernetzt ist, indem man die schaumförmigen, vernetzten Polymerisate mit mindestens 2 reaktive Gruppen aufweisenden Vernetzungsreagenzien imprägniert, die beim Erhitzen auf Temperaturen oberhalb von 70°C mit den im Hydrogel enthaltenen Säuregruppen reagieren.

6. Verfahren zur Herstellung von wasserabsorbierenden, schaumförmigen, vernetzten Polymerisaten, dadurch gekennzeichnet, daß man eine polymerisierbare wäßrige Mischung aus

(a) 10 bis 80 Gew.-% Säuregruppen enthaltenden monoethylenisch ungesättigten Monomeren, die zu mindestens 50 Mol% neutralisiert sind,

(b) gegebenenfalls bis zu 50 Gew.-% anderen monoethylenisch ungesättigten Monomeren,

(c) 0,001 bis 5 Gew.-% Vernetzer,

(d) gegebenenfalls mindestens einem Polymerisationinitiator,

(e) 0,1 bis 20 Gew.-% mindestens einem Tensid,

(f) gegebenenfalls mindestens einem Lösevermittler und

(g) gegebenenfalls Verdickern, Schaumstabilisatoren,Polymerisationsreglern, Füllstoffen und/oder Zellkeimbildnern

in einer ersten Verfahrensstufe durch Dispergieren von feinen Blasen eines gegenüber Radikalen inerten Gases schäumt und den so erhaltenen Schaum in einer zweiten Verfahrensstufe unter Bildung eines schaumförmigen Hydrogels polymerisiert und den Wassergehalt des schaumförmigen Hydrogels auf 1 bis 45 Gew.-% einstellt.

7. Verfahren nach Anspruch 6, dadurch gekennzeichnet, daß man als Monomere

(a) Acrylsäure, Methacrylsäure, Vinylsulfonsäure, Acrylamidopropansulfonsäure oder deren Mischungen, und

(c) eine Mischung aus mindestens einem wasserlöslichen und mindestens einem wasserunlöslichen Vernetzer einsetzt.

8. Verfahren nach Anspruch 6 oder 7, dadurch gekennzeichnet, daß man als wasserlösliche Vernetzer (c) Acrylsäure- und Methacrylsäureester von mindestens zweiwertigen Alkoholen oder Methylenbisacrylamid einsetzt.

9. Verfahren nach Anspruch 6, dadurch gekennzeichnet, daß man als Tenside (e) Additionsprodukte von Ethylenoxid und/oder Propylenoxid an mindestens 10 C-Atome enthaltende Alkohole einsetzt, wobei die Additionsprodukte pro Mol Alkohol 3 bis 200 Mol Ethylenoxid und/oder Propylenoxid angelagert enthalten.

10. Verfahren nach Anspruch 6, dadurch gekennzeichnet, daß man als Tenside (e) Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäuren oder von Alkylphenolethersulfaten einsetzt.

**11.** Verfahren nach Anspruch 6, dadurch gekennzeichnet, daß man als Tenside (e) Quarternisierungsprodukte von tert. Aminen oder Aminestern einsetzt, die mindestens einen $C_{10}$- bis $C_{18}$-Alkylrest enthalten.

**12.** Verfahren nach Anspruch 6, dadurch gekennzeichnet, daß man als Verdicker wasserquellbare oder wasserlösliche synthetische oder natürliche Polymere einsetzt.

**13.** Verfahren nach Anspruch 6, dadurch gekennzeichnet, daß man als Verdicker pulverförmige Superabsorber einsetzt.

**14.** Verfahren nach Anspruch 6, dadurch gekennzeichnet, daß man als Stabilisatoren für die geschäumten wäßrigen Mischungen aliphatische Kohlenwasserstoffe einsetzt, deren Siedetemperatur oberhalb der Temperatur der wäßrigen Mischung während des Schäumens liegt.

**15.** Verwendung der wasserabsorbierenden, schaumförmigen, vernetzten Polymerisate nach den Ansprüchen 1 bis 5 in Sanitärartikeln, die zur Absorption von Körperflüssigkeiten eingesetzt werden, in Verbandmaterial zur Abdeckung von Wunden, als Dichtungsmaterial, als Bodenverbesserungsmittel, als Bodenersatzstoff und als Verpackungsmaterial.

**16.** Verwendung der wasserabsorbierenden, schaumförmigen, vernetzten Polymerisate nach den Ansprüchen 1 bis 5 in Form eines Pulvers mit einem mittleren Teilchendurchmesser von 150 μm bis 5 mm in Sanitärartikeln, die zur Absorption von Körperflüssigkeiten eingesetzt werden, in Verbandmaterial zur Abdeckung von Wunden, als Dichtungsmaterial, als Bodenverbesserungsmittel und als Bodenersatzstoff zum Kultivieren von Pflanzen.

**Claims**

**1.** A water-absorbing, predominantly open-celled expanded, crosslinked polymer obtainable by

(I) foaming a polymerizable aqueous mixture which comprises

(a) 10-80% by weight of monoethylenically unsaturated monomers which contain acidic groups and are at least 50 mol% neutralized,

(b) with or without up to 50% by weight of other monoethylenically unsaturated monomers,

(c) 0.001-5% by weight of crosslinkers,

(d) initiators,

(e) 0.1-20% by weight of at least one surfactant,

(f) with or without at least one solubilizer and

(g) with or without thickeners, foam stabilizers, polymerization regulators, fillers and/or cell nucleating agents,

where the foaming takes place by dispersing fine bubbles of a gas which is inert to free radicals, and

(II) polymerizing the foamed mixture to form an expanded hydrogel and adjusting the water content of the expanded polymer to 1-45% by weight.

**2.** A water-absorbing, expanded, crosslinked polymer as claimed in claim 1, wherein the polymerizable aqueous mixture employed comprises as

(a) monoethylenically unsaturated monomers which contain acidic groups and are at least 50 mol% neutralized,

(b) $C_2$-$C_{25}$-olefins, esters of monoethylenically unsaturated $C_3$-$C_5$-carboxylic acids and monohydric $C_2$-$C_{25}$-

alcohols, polyethylene glycol or polypropylene glycol monoesters of monoethylenically unsaturated $C_3$-$C_5$-carboxylic acids, vinyl esters of saturated carboxylic acids containing at least 4 carbon atoms, alkyl vinyl ethers with at least 2 carbon atoms in the alkyl group, alkyl-substituted styrenes, hydroxyalkyl esters of monoethylenically unsaturated $C_3$-$C_5$-carboxylic acids, N-vinyllactams, N-vinylimidazoles with 5 to 8 carbon atoms, and their salts and quaternization products, basic esters or amides of monoethylenically unsaturated $C_3$-$C_5$-carboxylic acids, and their salts and quaternization products.

3. A water-absorbing, expanded, crosslinked polymer as claimed in claim 1, wherein the polymerizable aqueous mixture employed consists of

(a) monoethylenically unsaturated monomers which contain acidic groups and are at least 50 mol% neutralized, and

(b) a mixture of at least one water-soluble and at least one water-insoluble crosslinker.

4. A water-absorbing, expanded, crosslinked polymer as claimed in claim 1 or 2, which comprises 0.1-10% by weight of an external plasticizer from the group of polyols, polyalkylene glycols and cationic surfactants.

5. A water-absorbing, expanded, crosslinked polymer as claimed in claim 1, wherein the entire surface is subsequently crosslinked by impregnating the expanded, crosslinked polymer with crosslinking reagents which have at least 2 reactive groups and react with the acidic groups present in the hydrogel on heating above 70°C.

6. A process for producing water-absorbing, expanded, cross-linked polymers, which comprises foaming a polymerizable aqueous mixture of

(a) 10-80% by weight of monoethylenically unsaturated monomers which contain acidic groups and are at least 50 mol% neutralized,

(b) with or without up to 50% by weight of other monoethylenically unsaturated monomers,

(c) 0.001-5% by weight of crosslinker,

(d) with or without at least one polymerization initiator,

(e) 0.1-20% by weight of at least one surfactant,

(f) with or without at least one solubilizer and

(g) with or without thickeners, foam stabilizers, polymerization regulators, fillers and/or cell nucleating agents,

in a first stage by dispersing fine bubbles of a gas which is inert to free radicals, and polymerizing the resulting foam in a second stage to form an expanded hydrogel, and adjusting the water content of the expanded hydrogel to 1-45% by weight.

7. A process as claimed in claim 6, wherein the monomers used are

(a) acrylic acid, methacrylic acid, vinylsulfonic acid, acrylamidopropanesulfonic acid or mixtures thereof, and

(c) a mixture of at least one water-soluble and at least one water-insoluble crosslinker.

8. A process as claimed in claim 6 or 7, wherein acrylic and methacrylic esters of at least dihydric alcohols or methylenebisacrylamide are used as water-soluble crosslinkers (c).

9. A process as claimed in claim 6, wherein adducts of ethylene oxide and/or propylene oxide and alcohols containing at least 10 carbon atoms, where the adducts contain from 3 to 200 mol of ethylene oxide and/or propylene oxide per mol of alcohol, are used as surfactants (e).

10. A process as claimed in claim 6, wherein alkali metal or ammonium salts of sulfuric acid half esters of adducts of ethylene oxide and/or propylene oxide and fatty alcohols, alkali metal or ammonium salts of alkylbenzenesulfonic acids or of alkylphenol ether sulfates are used as surfactants (e).

11. A process as claimed in claim 6, wherein quaternization products of tertiary amines or amine esters which contain at least one $C_{10}$-$C_{18}$-alkyl radical are used as surfactants (e).

12. A process as claimed in claim 6, wherein water-swellable or water-soluble synthetic or natural polymers are used as thickeners.

13. A process as claimed in claim 6, wherein superabsorbents in powder form are used as thickeners.

14. A process as claimed in claim 6, wherein aliphatic hydrocarbons whose boiling point is above the temperature of the aqueous mixture during the foaming are used as stabilizers for the foamed aqueous mixtures.

15. The use of the water-absorbing, expanded, crosslinked polymers as claimed in claims 1 to 5 in hygiene articles employed to absorb body fluids, in dressing material for covering wounds, as sealing material, as soil improver, as soil substitute and as packaging material.

16. The use of the water-absorbing, expanded, crosslinked polymers as claimed in claims 1 to 5 in the form of a powder with an average particle diameter of 150 $\mu$m to 5 mm in hygiene articles employed to absorb body fluids, in dressing material for covering wounds, as sealing material, as soil improver and as soil substitute for growing plants.

**Revendications**

1. Polymères réticulés, sous forme de mousse essentiellement à cellules ouvertes, absorbant l'eau, caractérisés en ce qu'ils peuvent être obtenus par

(I) moussage d'un mélange aqueux polymérisable contenant

(a) 10-80% en poids de monomères à insaturation monoéthylénique contenant des groupements acide, neutralisés pour au moins 50% en moles,
(b) éventuellement jusqu'à 50% en poids d'autres monomères à insaturation monoéthylénique,
(c) 0,001-5% en poids d'agents réticulants,
(d) des amorceurs,
(e) 0,1-20% en poids d'au moins un tensioactif,
(f) éventuellement au moins un agent de solubilisation et
(g) éventuellement des épaississants, des agents stabilisants la mousse, des agents régulant la polymérisation, des matières de charge et/ou des agents d'ensemencement,

où le moussage est effectué par dispersion de fines bulles d'un gaz inerte vis à vis des radicaux, et
(II) polymérisation du mélange en mousse pour former un hydrogel sous forme de mousse et réglage de la teneur en eau du polymère en mousse à 1-45% en poids.

2. Polymères réticulés, sous forme de mousse, absorbant l'eau, selon la revendication 1, caractérisés en ce que l'on utilise un mélange aqueux polymérisable contenant

(a) des monomères à insaturation monoéthylénique contenant des groupements acide, neutralisés pour au moins 50% en moles,
(b) des oléfines en $C_2$-$C_{25}$, des esters d'acides carboxyliques en $C_3$-$C_5$ à insaturation monoéthylénique et d'alcools monofonctionnels en $C_2$-$C_{25}$, des monoesters de polyéthylèneglycol ou de polypropylèneglycol avec des acides carboxyliques en $C_3$-$C_5$ à insaturation monoéthylénique, des esters vinyliques d'acides carboxyliques saturés contenant au moins 4 atomes de C, des alkylvinyléthers ayant au moins deux atomes de C dans le groupement alkyle, des styrènes à substitution alkyle, des hydroxyalkylesters d'acides carboxyliques en $C_3$-$C_5$ à insaturation monoéthylénique, des n-vinyllactames, des N-vinylimidazole ayant de 5 à 8 atomes de C ainsi que leurs sels et leurs produits de quaternisation, des esters ou des amides basiques d'acides carboxyliques en $C_3$-$C_5$ à insaturation monoéthylénique ainsi que leurs sels et leurs produits de quaternisation.

**3.** Polymères réticulés, sous forme de mousse, absorbant l'eau, selon la revendication 1, caractérisés en ce que l'on utilise un mélange aqueux polymérisable contenant

(a) des monomères à insaturation monoéthylénique contenant des groupements acide, neutralisés pour au moins 50% en moles,
(b) un mélange d'au moins un agent réticulant soluble dans l'eau et d'au moins un agent réticulant insoluble dans l'eau.

**4.** Polymères réticulés, sous forme de mousse, absorbant l'eau, selon la revendication 1 ou 2, caractérisés en ce qu'ils contiennent 0,1-10% en poids d'un agent plastifiant externe choisi dans le groupe formé par les polyols, les polyalkylèneglycols et les tensioactifs cationiques.

**5.** Polymères réticulés, sous forme de mousse, absorbant l'eau, selon la revendication 1, caractérisés en ce que la surface entière est post-réticulée, les polymères réticulés sous forme de mousse étant imprégnés avec des réactifs de réticulation comportant au moins deux groupements réactifs, qui réagissent avec les groupements acide de l'hydrogel lorsqu'on chauffe à des températures supérieures à 70°C.

**6.** Procédé de préparation de polymères réticulés, sous forme de mousse, absorbant l'eau, caractérisés en ce que, dans une première étape, on fait mousser un mélange aqueux polymérisable contenant

(a) 10-80% en poids de monomères à insaturation monoéthylénique contenant des groupements acide, neutralisés pour au moins 50% en moles,
(b) éventuellement jusqu'à 50% en poids d'autres monomères à insaturation monoéthylénique,
(c) 0,001-5% en poids d'agents réticulants,
(d) éventuellement au moins un amorceur de polymérisation,
(e) 0,1-20% en poids d'au moins un tensioactif,
(f) éventuellement au moins un agent de solubilisation et
(g) éventuellement des épaississants, des agents stabilisants la mousse, des agents régulant la polymérisation, des matières de charge et/ou des agents d'ensemencement,

par dispersion de fines bulles d'un gaz inerte vis à vis des radicaux, et, dans une deuxième étape, on polymérise la mousse ainsi obtenue pour former un hydrogel sous forme de mousse et on règle la teneur en eau de l'hydrogel sous forme de mousse à 1-45% en poids.

**7.** Procédé selon la revendication 6, caractérisé en ce qu'on utilise en tant que monomères

(a) de l'acide acrylique, de l'acide méthacrylique, de l'acide vinylsulfonique, de l'acide acrylamidopropanesulfonique ou leurs mélanges, et
(c) un mélange d'au moins un agent réticulant soluble dans l'eau et d'au moins un agent réticulant insoluble dans l'eau.

**8.** Procédé selon la revendication 6 ou 7, caractérisé en ce qu'on utilise, en tant qu'agent réticulant soluble dans l'eau (c) des esters de l'acide acrylique et de l'acide méthacrylique avec des alcools au moins bifonctionnels ou du méthylènebisacrylamide.

**9.** Procédé selon la revendication 6, caractérisé en ce qu'on utilise en tant qu'agent tensioactif (e) des produits d'addition d'oxyde d'éthylène et/ou d'oxyde de propylène sur des alcools contenant au moins 10 atomes de C, les produits d'addition contenant 3-200 moles d'oxyde d'éthylène et/ou d'oxyde de propylène par mole d'alcool.

**10.** Procédé selon la revendication 6, caractérisé en ce qu'on utilise en tant qu'agent tensioactif (e) des sels de métaux alcalins ou d'ammonium d'hémiesters d'acide sulfurique et de produits d'addition d'oxyde d'éthylène et/ou d'oxyde de propylène sur des alcools gras, des sels de métaux alcalins ou d'ammonium d'acides alkylbenzène-sulfonique ou d'éthersulfates d'alkyklphénol.

**11.** Procédé selon la revendication 6, caractérisé en ce qu'on utilise en tant qu'agent tensioactif (e) des produits de quaternisation d'amines tertiaires ou d'esters d'amines, contenant au moins un reste alkyle en $C_{10}$-$C_{18}$

**12.** Procédé selon la revendication 6, caractérisé en ce qu'on utilise en tant qu'épaississant des polymères naturels ou synthétiques solubles dans l'eau ou susceptibles de gonfler dans l'eau.

13. Procédé selon la revendication 6, caractérisé en ce qu'on utilise en tant qu'épaississant un superabsorbant sous forme de poudre.

14. Procédé selon la revendication 6, caractérisé en ce que l'on utilise, en tant qu'agents stabilisants pour les mélanges aqueux en mousse, des hydrocarbures aliphatiques dont le point d'ébullition est situé au dessus de la température du mélange aqueux au cours du moussage.

15. Utilisation de polymères réticulés, sous forme de mousse, absorbant l'eau, selon l'une quelconque des revendications 1 à 5, dans des articles d'hygiène utilisés pour l'absorption de liquides corporels, dans des bandages pour recouvrir des blessures, en tant que matériau d'étanchéité, en tant qu'agent d'amélioration des sols, en tant qu'agent de remplacement des sols et en tant que matériau d'emballage.

16. Utilisation de polymères réticulés, sous forme de mousse, absorbant l'eau, selon l'une quelconque des revendications 1 à 5, sous forme d'une poudre ayant un diamètre moyen de particules de 150 μm à 5 mm dans des articles d'hygiène utilisés pour l'absorption de liquides corporels, dans des bandages pour recouvrir des blessures, en tant que matériau d'étanchéité, en tant qu'agent d'amélioration des sols et en tant qu'agent de remplacement des sols pour la culture des plantes.